# EUROPEAN PATENT APPLICATION

(11) **EP 4 109 397 A1**
(43) Date of publication of application: **28.12.2022**
(21) Application number: 21181026.2
(22) Date of filing: 23.06.2021
(51) Int. Cl.: G06T 7/00, G06T 7/60

(54) **IDENTIFYING STENT DEFORMATIONS**

(71) Applicant: Koninklijke Philips N.V., 5656 AG Eindhoven (NL)
(72) Inventor: WISSEL, Tobias, 5656 AE Eindhoven (NL)
(74) Representative: Philips Intellectual Property & Standards

(57) **Abstract**

A system (100) for identifying deformations of a deployed stent, is provided. The system includes one or more processors (110) configured to: receive (S110) X-ray image data representing one or more X-ray images (120) of a deployed stent (130) within a lumen (140), the stent including a plurality of stent struts (150); analyse (S120) the X-ray image data to determine a distribution of the stent struts (150) along an axis (160) of the lumen (140); and identify (S130) one or more longitudinally-deformed portions (170, 180) of the stent based on a density of the determined distribution of the stent struts (150) along the axis (160) of the lumen (140).

## Description

### Technical Field

The present disclosure relates to identifying deformations of a deployed stent. A system, a computer-implemented method, and a computer program product, are disclosed.

### Background

In the medical field, stents are routinely inserted into lumens in order to keep passageways open. For example, stents are often inserted into vascular, coronary, biliary, bronchial, urinary, and other lumens. Stents typically include a tubular mesh that is formed from a flexible material such as a metal or a polymer. Prior to deployment, a stent is maintained in a radially-compressed state in order to facilitate its insertion into the lumen. During deployment, the stent is expanded radially such that the tubular mesh supports the inner wall of the passageway. Stents are typically self-expanding, or balloon-expandable. Stents are typically deployed by a physician under the guidance of an external X-ray imaging system.

By way of an example, stents are often used to treat atherosclerotic lesions. In such procedures, a stent is delivered minimally invasively into a vessel via a catheter of a stent deployment device. The stent is typically specified with a length that is sufficient to cover the stenosed, i.e. narrowed, portion of the vessel. The stent is deployed so as to open-up the stenosed portion, and also acts to stabilise plaques that are prone to rupture. A further consideration is often that the stent length should not cover, and consequently block, any bifurcations in the vessel. An X-ray imaging system is used to deploy the stent in the target location, often with the support of an intraluminal imaging system such as an intravascular ultrasound "IVUS", or optical coherence tomography "OCT", imaging system.

An important parameter in the specification of a stent is therefore its post-deployment length, i.e. its deployed length. Further stent parameters that may be specified include the stent material. Stents may be formed from various metal or polymer materials including nitinol, stainless steels, nylon and polyurethane. Stents may also be specified as bare, or coated. One type of coating is a drug coating that is used in a drug-eluting stent to administer a drug to an atherosclerotic vessel wall.

A deployed stent typically has a nominal deployed length. This is defined as the length of the stent in the absence of any longitudinal compressive or extensive deformations. A building block of a stent is a stent strut, which has a shape that is repeated along the length of the stent to define its tubular mesh. In a stent with its nominal deployed length, the stent struts are distributed periodically along the length of the stent, or in other words, with a constant density along the axis of the lumen.

However, due to their flexible nature, various factors can affect the deployed length of stents. For example, the rate at which the stent is deployed may affect whether longitudinal compressive or extensive deformations are introduced to the stent. The deployed length of a self-expanding stent may also be controlled to some extent by applying a compressive force to the stent via the proximal end of the plunger of the stent deployment device whilst withdrawing the stent's outer catheter.

As a result, the deployed length of a stent can vary between procedures. The deployed length may consequently be insufficient to cover the intended portion of the lumen, or alternatively be so long that it unintentionally overlaps with another portion of the lumen such as a bifurcation. Such length variations may also impact the mechanical performance of a stent. For example, longitudinal extensions of a stent reduce its radial stability, and may lead to a narrowing of the stent over time. Such longitudinal extensions have also been suggested as being a factor in the failure of drug-eluting stents.

Consequently, there is a need for improvements in identifying deformations in deployed stents.

### Summary

According to one aspect of the present disclosure, a system for identifying deformations of a deployed stent, is provided. The system includes one or more processors configured to:
- receive X-ray image data representing one or more X-ray images of a deployed stent within a lumen, the stent including a plurality of stent struts;
- analyse the X-ray image data to determine a distribution of the stent struts along an axis of the lumen; and
- identify one or more longitudinally-deformed portions of the stent based on a density of the determined distribution of the stent struts along the axis of the lumen.

In so doing, the system facilitates a physician to determine whether a stent has been deployed correctly. Further aspects, features, and advantages of the present disclosure will become apparent from the following description of examples, which is made with reference to the accompanying drawings.

### Brief Description of the Drawings

Fig. 1 illustrates an example of an X-ray image 120 including a deployed stent 130 with two longitudinally-deformed portions.
Fig. 2 illustrates an example of a system 100 for identifying deformations of a deployed stent, in accordance with some aspects of the disclosure.
Fig. 3 illustrates an example of a method of identifying deformations of a deployed stent, in accordance with some aspects of the disclosure.
Fig. 4 illustrates an example of a displayed X-ray image 120 including a stent 130 and wherein longitudinally-deformed portions of the stent are identified, in accordance with some aspects of the disclosure.
Fig. 5 illustrates various examples of deformed stents 130 within a lumen 140: (A) un-deformed stent with expected distribution of stent struts 150 along the axis 160 of lumen 140, (B) stent with central longitudinally-compressed portion 170, (C) radially deformed stent with expected distribution of stent struts 150 along the axis 160 of lumen 140, and (D) curved stent with central longitudinally-compressed portion 170 and outer longitudinally-extended portions 180, in accordance with some aspects of the disclosure.
Fig. 6 illustrates an example of a method of identifying deformations of a deployed stent that includes inputting an X-ray image 120 into a neural network 200, in accordance with some aspects of the disclosure.

### Detailed Description

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer implemented method, and in a computer program product, in a corresponding manner.

In the following description, reference is made to a system for identifying deformations of a deployed stent. Reference is made to examples in which a vascular stent is deployed in a blood vessel in order to treat an atherosclerotic lesion. It is, however, to be appreciated that the system may also be used to identify deformations in other types of stents, including coronary, biliary, bronchial, urinary, and other types of stents, and which may consequently, and as appropriate, be inserted into other lumens within the anatomy.

It is also to be appreciated that the stent may be deployed manually, or with the assistance of a robotic controller. In some instances, the stent deployment device is manipulated manually by the physician directly handling a portion of the device as it exits the patient. In other instances the physician manually controls a robotic controller that provides a mechanical interface with the device. In yet other instances, a robotic controller automatically manipulates the stent deployment device within the anatomy. Robotic controllers that use rollers and/or fingers that grip the stent deployment device in order to perform translational and/or rotational operations in order to insert and to deploy stents, are known for this purpose.

Reference is also made herein to the use of X-ray image data. In this respect it is to be appreciated that the X-ray image data may be generated by various types of X-ray imaging systems. For example, the X-ray image data may be generated by a planar X-ray imaging system that generates planar X-ray images, or a volumetric X-ray imaging system that generates volumetric X-ray images. Planar X-ray imaging systems typically include a support arm such as a so-called "C-arm", or an "O-arm", that supports an X-ray source-detector arrangement. Planar X-ray imaging systems may alternatively include a support arm with a different shape to these examples. Planar X-ray imaging systems typically generate planar X-ray images with the support arm held in a static position with respect to an imaging region during the acquisition of image data. By contrast, volumetric X-ray imaging systems typically generate image data whilst rotating, or stepping, an X-ray source-detector arrangement around an imaging region, and subsequently reconstruct the image data obtained from multiple rotational angles into a volumetric image. Examples of volumetric X-ray imaging systems include computed tomography "CT" imaging systems, cone beam CT "CBCT" imaging systems, and spectral CT imaging systems.

It is noted that the methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, the internet, or the cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD

As mentioned above, an important parameter in the specification of a stent is its deployed length. In the absence of any longitudinal compressive or extensive deformations, a deployed stent has a nominal deployed length. However, due to their flexible nature, various factors can affect the deployed length of stents. As a result, the deployed length of a stent may vary between procedures.

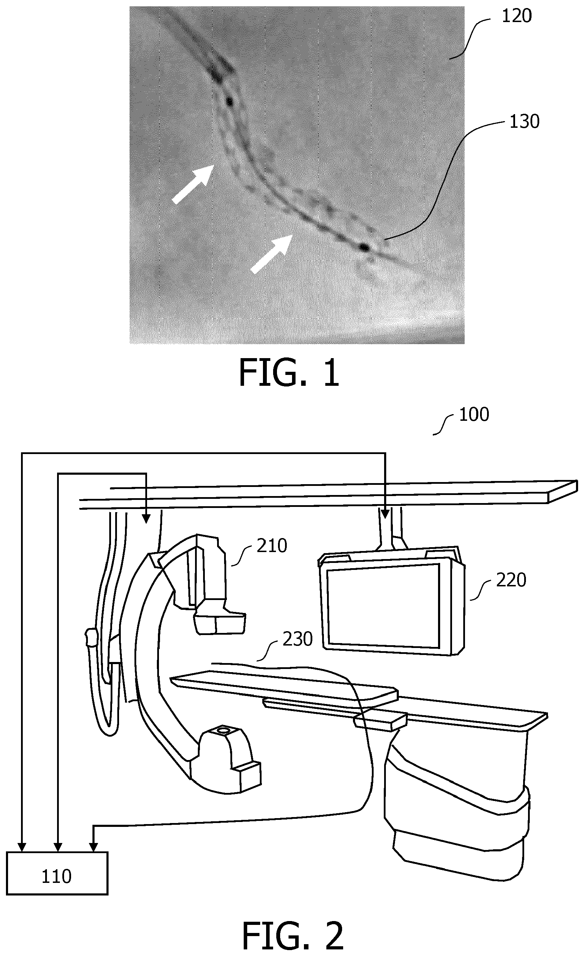

By way of an example, Fig. 1 illustrates an example of an X-ray image 120 including a deployed stent 130 with two longitudinally-deformed portions. The longitudinally-deformed portions in Fig. 1 are indicated by arrows, and are longitudinally-compressed portions that may have been introduced during deployment of the stent 130, or subsequent to its deployment. As mentioned above, the mechanical performance of the stent 130, and potentially also its drug eluting performance, may be affected by such longitudinal deformations.

Fig. 2 illustrates an example of a system 100 for identifying deformations of a deployed stent, in accordance with some aspects of the disclosure. The system 100 includes one or more processors 110 configured to:
- receive S110 X-ray image data representing one or more X-ray images 120 of a deployed stent 130 within a lumen 140, the stent including a plurality of stent struts 150;
- analyse S120 the X-ray image data to determine a distribution of the stent struts 150 along an axis 160 of the lumen 140; and
- identify S130 one or more longitudinally-deformed portions 170, 180 of the stent based on a density of the determined distribution of the stent struts 150 along the axis 160 of the lumen 140.

In so doing, the system 100 facilitates a physician to determine whether a stent has been deployed correctly. The system may be used during deployment of the stent, and also post-deployment. For example, it may be used during, or shortly after a stent has been deployed in order to verify the stent's deployment. It may also be used some days, weeks, or months after deployment to identify whether the stent has become longitudinally-deformed over time. The physician may use this information for various purposes. For example, the physician may use it to correct a current deployment of the stent by redeploying it, or to deploy the stent with increased radial support in a calcified portion of the lumen by introducing a longitudinal compression to this portion of the stent, or to undertake a further clinical investigation using e.g. an intravascular imaging "IVUS" or optical coherence tomography "OCT" imaging device, or to insert another stent.

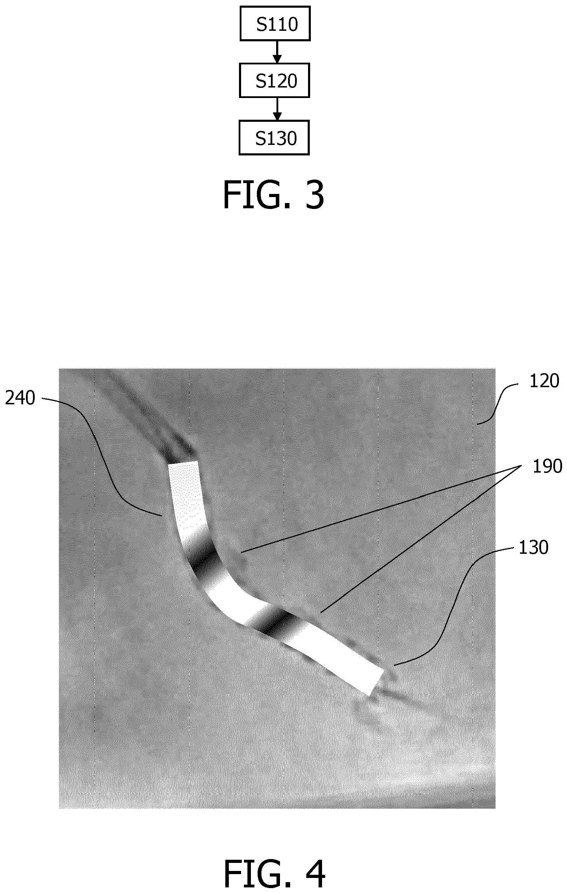

Fig. 3 illustrates an example of a method of identifying deformations of a deployed stent, in accordance with some aspects of the disclosure. The operations performed in the method illustrated in Fig. 3 correspond to the operations mentioned above that are performed by the processor(s) 120 of the system 100 in Fig. 2. The method illustrated in Fig. 3 may be implemented by a computer. The method illustrated in Fig. 3 may also be provided in the form of instructions of a computer program product, or on a computer readable storage medium.

With reference to Fig. 2 and Fig. 3, in the operation S110, X-ray image data is received. The X-ray image data may be received via any form of data communication, including wired, optical, and wireless communication. By way of some examples, when wired or optical communication is used, the communication may be effected using an electrical cable or optical fibre, and when wireless communication is used, the communication may be effected using RF or infrared signals. In one example, the X-ray image data is received from an X-ray imaging system 210, as illustrated in Fig. 2. Various types of X-ray imaging systems may be used to generate the X-ray image data, as mentioned above. The X-ray image data may alternatively be received from another source, such as for example a computer readable storage medium, the internet, the cloud, and so forth.

The X-ray image data that is received in the operation S110 represents one or more X-ray images 120 of a deployed stent 130 within a lumen 140. In general, the X-ray image data may represent a single X-ray image, or a temporal sequence of X-ray images. If a temporal sequence of X-ray images is provided, the X-ray images may be live X-ray images in the sense that the X-ray images are generated instantaneously before being received in the operation S110. Alternatively, the temporal sequence of X-ray images may have been generated some seconds, minutes, hours, or even days, or a longer period, beforehand. The lumen 140 represented in the X-ray images 130 may be a lumen within the vasculature, or indeed another lumen within the anatomy.

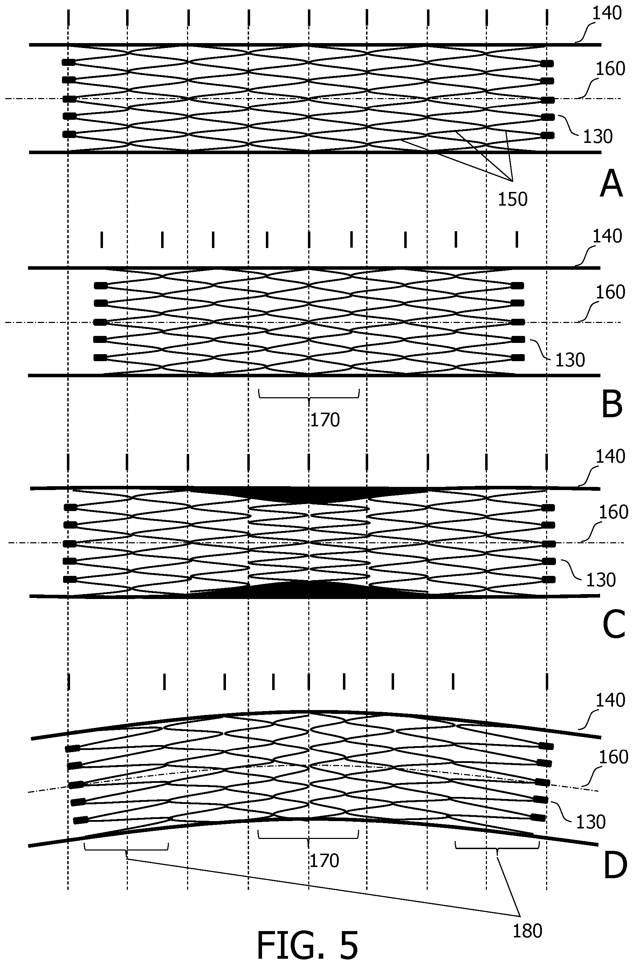

The stent in the X-ray image(s) 120 received in the operation S110 includes a plurality of stent struts 150. As mentioned above, a building block of a stent is a stent strut, and which has a shape that is repeated along the length of the stent to define its tubular mesh. Fig. 5 illustrates various examples of deformed stents 130 within a lumen 140: (A) un-deformed stent with expected distribution of stent struts 150 along the axis 160 of lumen 140, (B) stent with central longitudinally-compressed portion 170, (C) radially deformed stent with expected distribution of stent struts 150 along the axis 160 of lumen 140, and (D) curved stent with central longitudinally-compressed portion 170 and outer longitudinally-extended portions 180, in accordance with some aspects of the disclosure. The interval between adjacent stent struts is illustrated in each of Fig. (A) - (D) via the vertical lines above each figure. The dashed lines serve as a reference for the intervals between adjacent struts in the un-deformed stent of Fig. 5 (A).

As compared to Fig. 5 (A), the stent in Fig. 5 (B) has a central longitudinally-compressed portion 170, which may for example the result of a longitudinal compressive force during its deployment into the lumen 140. As compared to Fig. 5 (A), the stent in Fig. 5 (C) has the same longitudinal spacing of stent struts, and a radial compression, the latter resulting from the central stenosed region of the lumen 140. As compared to Fig. 5 (A), the stent in Fig. 5 (D) is curved and includes both a central longitudinally-compressed portion 170 and two outer longitudinally-extended portions 180. The deformations in the stent in Fig. 5 (D) may arise from the difficulties in deploying the stent in a curved lumen. As illustrated in Fig. 5, longitudinal compressions and extensions result in variations in length of the stent 140, and may also result in variations in a stent's radial stability, or in other words, its ability to keep a lumen open.

With reference to Fig. 5 (A), in this example, each stent strut 150 of the stent 130 has a sinusoidal shape. Each sinusoidal shape represents an annular section of the stent 130. The sinusoidal shape in each annular section is repeated along the length of the stent 130. The stent 130 illustrated in Fig. 5 (A) has no longitudinal deformations. Thus, the stent in Fig. 5 (A) has its nominal deployed length. The stent struts 150 in Fig. 5 (A) are also distributed periodically along the length of the stent, or in other words, with a constant density, i.e. struts per unit of length, along the axis of the lumen 140. In the presence of external forces, the shape of the stent struts 150 may be deformed as compared to the stent illustrated in Fig. 5 (A). For example, the stent 130 may be longitudinally-compressed or longitudinally-extended as illustrated in Fig. 5 (B) and Fig. 5 (D) respectively, as described in more detail below. The stent may also be radially compressed, as illustrated in Fig. 5 (C). In general, stents may also have struts that have different shapes to the example sinusoidal shape illustrated in Fig. 5, such as for example zigzag, diamond, and other shapes.

Returning to Fig. 3, in the operation S120, the X-ray image data is analysed in order to determine a distribution of the stent struts 150 along an axis 160 of the lumen 140. In this respect, the use of various techniques is contemplated.

In a first example technique, an edge filter is applied to the X-ray image(s) 120 and a stent model is fitted to the filtered X-ray image(s) 120. In this technique, the X-ray image(s) 120 of the deployed stent 120, is initially filtered for edges or lines in order to isolate or highlight the stent struts. A filter such as a Canny edge detector, or another pre-defined convolutional (high-pass) filter may be used for this purpose. The filter may be designed such that it emphasises edges that are disposed along a direction that is orthogonal to the axis 160 of the lumen 140. In one example, one or a bank of Garbor filter(s) may be used for this purpose.

Having determined the distribution of the stent struts along the axis of the lumen by highlighting the stent struts in this manner, a density of the distribution of the stent struts 150 along the axis 160 of the lumen 140 can be estimated by fitting a model of the stent, such as a 2D projected model of the stent, to the edge-filtered X-ray image(s). In order to do this, a 3D model of the stent may for example be provided, e.g. by a CAD model, and projected onto a plane in order to provide the projected 2D projected model. The 2D projected model of the stent may for example be provided as a projection image of a 3D tubular stent including multiple struts 150, and which have a degree of freedom to slide along the axis of the stent in order to mimic longitudinal compression and extension. During fitting, the stent model is oriented along the vessel and scaled based on the X-ray image resolution. The positions of the struts along the axis of the stent in the 2D projected model are then optimised until there is a fit between the model and the edge-filtered X-ray image(s). Longitudinally-deformed portions 170, 180 of the stent may be identified based on the deformations of the fitted model in relation to a model of the stent with its nominal deployed length, or in other words, the stent as illustrated in Fig. 5 (A).

Thus, in this first example technique, the one or more processors 110 are configured to analyse the X-ray image data in the operation S120 by:
- applying a spatial filter to the one or more X-ray images 120, and estimating a density of the distribution of the stent struts 150 along the axis 160 of the lumen 140 by fitting a stent model to the filtered one or more X-ray images.

In a second example technique, image templates are applied to the X-ray image(s) 120 to determine a distribution of the stent struts 150 along an axis 160 of the lumen 140. In this example, image templates that represent the stent struts, such as for example stripe-like image templates of known strut density, are applied to the X-ray image(s) 120 in order to identify a closest match between the image templates and the X-ray image(s) at positions along the axis of the stent in the X-ray image(s) 120. The closest match may be determined by computing the value of a cross correlation function between each template and the X-ray image(s) 120. Each image template exhibits the texture characteristic of an X-ray projection of a predetermined strut density along a portion of the axis of the stent. Since the image templates have a known strut density, longitudinally-deformed portions 170, 180 of the stent may be identified based on deviations between the strut density as determined from the templates, and the strut density of the stent with its nominal deployed length, or in other words, the stent as illustrated in Fig. 5 (A).

Thus, in this second example technique, the one or more processors 110 are configured to analyse the X-ray image data in the operation S120 by:
- estimating a density of the distribution of the stent struts 150 along the axis 160 of the lumen 140 by applying one of more image templates representing a predetermined distribution of stent struts 150, to the one or more X-ray images 120.

In a third example technique, the one or more processors 110 analyse the X-ray image data in the operation S120 by inputting the one or more X-ray images 120 into a neural network 200 trained to predict a stent strut density distribution from inputted X-ray images 120.

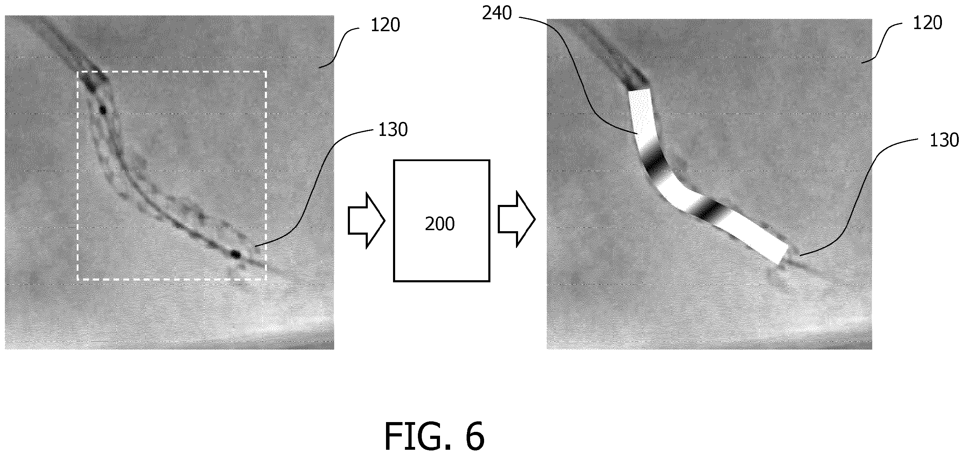

The neural network may for example be a regression-type convolutional neural network "CNN" that assigns a scalar value representing the strut density, or similarly a deployed strut interval, to locations along the stent's axis. Fig. 6 illustrates an example of a method of identifying deformations of a deployed stent that includes inputting an X-ray image 120 into a neural network 200, in accordance with some aspects of the disclosure. In this example, the filters of the regression-type CNN 200 learn to be appropriate filters for detecting image characteristics of the strut density. In this example, the deformations are again determined with respect to the strut density of the stent with its nominal deployed length, or in other words, the stent as illustrated in Fig. 5 (A). The neural network 200 may be specific to, or conditioned on, a particular type of stent.

The neural network may be trained to predict a stent strut density distribution from inputted X-ray images 120, by:
- receiving X-ray training image data representing a plurality of X-ray images 120 including a stent 130;
- receiving ground truth data representing a labelled strut density along an axis of the stent 130;
- for a plurality of the X-ray images 120 inputting the X-ray training image data into the neural network; and
- adjusting parameters of the neural network based on a loss function representing a difference between a predicted strut density along an axis of the stent 130 that is predicted by the neural network, and the ground truth data for the stent 130.

The training data used for training the neural network 200 may for example be provided by high resolution images of in-vivo stents, forward simulated projections of, for instance CAD, stent models, or phantoms. The ground truth data may be proved by manual analysis of the training data, or based on a knowledge of the stent strut density distribution in the phantom. Alternatively, the training data and corresponding ground truth data may be provided by simulations using a projected stent model.

In general, the above three example techniques may be applied to a single X-ray image 120, and also applied to multiple X-ray images 120. When multiple X-ray images 120 are available, each X-ray image, or a selection of the X-ray images, such as for example every N^{th} image, wherein N is an integer, may be processed according to the relevant technique. Processing every N^{th} image reduces the processing burden. In situations where the multiple X-ray images 120 represent a temporal sequence, there is likely to be motion between the individual X-ray images. Moreover, some of the X-ray images may be better than others. In order to compensate for these effects, a temporal sequence of X-ray images may be processed such that the distribution of the stent struts 150 for a current X-ray image is determined based on the current X-ray image and also on one or more historic X-ray images in the temporal sequence. For example, the distribution of the stent struts 150 for a current X-ray image may be determined by averaging the distributions obtained for the current X-ray image and one or more historic X-ray images. It is also contemplated to disregard outlier distributions. For example, a distribution obtained for a current X-ray image may be disregarded if the distribution of the stent struts 150 obtained for the current X-ray image differs significantly from the distribution of the stent struts 150 obtained for one or more historic X-ray images in the temporal sequence. In so doing, the reliability of the determined distribution of the stent struts 150 may be improved.

In the above three example techniques, it is assumed that the X-ray image resolution of the X-ray image(s) 120 is known in order to fit a stent model to the filtered X-ray image(s) 120, or in order to apply the image templates to the X-ray image(s) 120, or when inputting the one or more X-ray images 120 to the neural network 200, and to thereby determine the density of the distribution of the stent struts 150 along the axis 160 of the lumen 140. In general, the X-ray image resolution may be expressed as a number of X-ray image pixels per real-world unit of length, such as pixels per centimetre, for example. The X-ray image resolution may in general be known from a calibration of the X-ray imaging system. A calibration may for example be provided for positions in a reference imaging plane passing through the isocenter of the X-ray support arm and which is parallel to the plane of the X-ray detector. Magnification effects occurring in imaging planes that are displaced normally with respect to the reference imaging plane may be accounted-for by modelling. A model may for example assume a displacement of the imaging plane with respect to the reference imaging plane, or alternatively, an estimate of this displacement may be inputted into the model by a user. Accurate measurements of the density of the distribution of the stent struts 150 can be obtained in this manner using such a calibration if the stent lies in a plane that is parallel to the X-ray detector. However, if the stent lies in a plane that is not parallel to the X-ray detector, foreshortening may impact the X-ray image resolution.

In one example, compensation for both foreshortening and magnification is provided by including in the X-ray image(s) 120 an interventional device that includes fiducial markers having a predetermined separation. Interventional devices such as stent deployment devices often include such fiducial markers. The fiducial markers may for example be disposed at one-centimetre intervals along the interventional device. The fiducial markers include a radiopaque material such as gold or platinum and are therefore visible in X-ray images. Moreover, since the interventional device is typically disposed within the same lumen as the stent, its orientation is matched to that of the stent, and thus its fiducial markers may be used to provide a scale factor for correcting the X-ray image resolution. Thus, in this example, the one or more X-ray images 120 represented in the X-ray image data include an interventional device comprising a plurality of fiducial markers having a predetermined separation; and the one or more processors 110 are further configured to:
- determine a scale factor for the one or more X-ray images 120 based on a measured separation between the plurality of fiducial markers in the one or more X-ray images 120, and the predetermined separation; and
- determine the density of the determined distribution of the stent struts 150 along the axis 160 of the lumen 140 based on the scale factor.

The scale factor may for example be computed as a number of X-ray image pixels per centimetre by dividing the number of pixels separating two or more of the fiducial markers in the X-ray image(s) 120 by the relevant inter-marker separation in real-world units.

Returning to Fig. 3, in the operation S130, one or more longitudinally-deformed portions 170, 180 of the stent are identified based on the density of the determined distribution of the stent struts 150 along the axis 160 of the lumen 140. The longitudinally deformed portions may include one or more longitudinally-compressed portions of the stent 130 and/or one or more longitudinally-extended portions of the stent 130. The deformations are, as mentioned above, determined with respect to the strut density of the stent with its nominal deployed length, or in other words, the stent as illustrated in Fig. 5 (A).

The identification of the longitudinally-deformed portions may take various forms. In one example, the one or more processors 110 identify the one or more longitudinally deformed portions of the stent by: displaying the one or more X-ray images 120, and indicating, in the displayed one or more X-ray images 120, the one or more longitudinally-deformed portions of the stent 130. Fig. 4 illustrates an example of a displayed X-ray image 120 including a stent 130 and wherein longitudinally-deformed portions of the stent are identified, in accordance with some aspects of the disclosure.

In Fig. 4, the locations of the longitudinally-compressed portions of the stent 130 are identified by overlaying the stent 130 in the X-ray image(s) 120 with a trace 240, and encoding the compressed portions of the stent 130 on the trace 240 with a marker 190. A marker in the form of a dark region is illustrated on the trace 240 in Fig. 4. Similarly, longitudinal extensions may be encoded on the trace 240 with a marker in the form of gap in the trace 240. In another example, longitudinal deformations may be encoded on the trace 240 using a marker in the form or a colour, for example encoding longitudinal compressions with a marker having a red colour and a saturation that depends on the magnitude of the compression. Longitudinal extensions may likewise be encoded with a marker having a blue colour and a saturation that depends on the magnitude of the extension. A marker with a green colour may be used to represent neutral regions where there is neither longitudinal compression nor extension and the stent struts have a density corresponding to the nominal deployed length.

Thus, in one example, the one or more processors 110 identify the one or more longitudinally-deformed portions of the stent 130 by:
- comparing the density of the determined distribution of the stent struts 150 along the axis 160 of the lumen 140, to one or more threshold density values; and
- assigning a marker 190 indicative of the one or more threshold density values to one or more corresponding portions of the stent 130.

The threshold density value may for example correspond to 40 percent compression, 20 percent compression, 10 percent compression, 10 percent extension, 20 percent extension, and 40 percent extension as compared to the strut density when the stent has the nominal deployed length. Alternative threshold density values to this example may alternatively be used, and the threshold density values may alternatively represent absolute values rather than a proportional change in strut density. Absolute threshold density values may be measured as a number of stent struts per centimetre, for example. In this example, having determined a density of the distribution of the stent struts 150 along the axis 160 of the lumen 140 as described in the three example techniques above, the density values of the determined distribution of the stent struts 150 along the axis 160 of the lumen 140 are compared to the threshold density values, and assigned a marker. Markers that indicate the strut density by way of a colour, or a saturation of a colour, may be used as described above in order to indicate longitudinal deformations along the length of the stent. The marker may be displayed in the X-ray image(s).

In one example, the one or more processors 110 compute the density of the determined distribution of the stent struts 150 along the axis 160 of the lumen 140 in relation to an expected density for the stent 130; and identify the one or more longitudinally-deformed portions of the stent 130, by displaying the computed density as a proportion of the expected density. The expected density may be the strut density when the stent has the nominal deployed length.

In another example, the actual length of the stent may be determined, and the one or more longitudinally-deformed portions 170, 180 of the stent are identified in the operation S130 by displaying the actual length as a proportion of an expected length of the stent 130. In this example, the one or more processors 120 compute the density of the determined distribution of the stent struts 150 along the axis 160 of the lumen 140. Moreover, an actual length of the stent 130 is determined based on the density. The actual length may be expressed as a number of pixels of the displayed X-ray image(s) 120, or as a length in real-world units such as centimetres. The actual length may be determined by integrating the strut density along the length of the stent.

In this example, the one or more processors 120 also identify the one or more longitudinally-deformed portions of the stent 130, by displaying the actual length as a proportion of an expected length of the stent 130. This may be displayed as a numerical value, for example by displaying "10 percent extension", or graphically, for example using a colour to represent the magnitude of the extension or compression as described above. In this example, the actual length, may be displayed as a numerical value, for example in real-world units such as centimetres, or as a number of pixels of the displayed X-ray image(s) 120.

In some examples, data representing one or more of: the actual length of the stent 130, the distribution of the stent struts 150 along an axis 160 of the lumen 140, and the density of the determined distribution of the stent struts 150 along the axis 160 of the lumen 140, may also be stored to a computer readable storage medium. This data may for example be stored as part of a medical report on the deployed stent. A physician may subsequently refer-back to this data at a future point in time in order to determine whether the stent has undergone any changes. The physician may therefore use this data to identify potential risk areas, or to assist in a later diagnosis.

In some examples, in the operation S120, the X-ray image data is only analysed within a defined extent of the X-ray image(s) 120. An example of a defined extent is illustrated by way of the bounding box with a dashed outline in Fig. 6. The extent may be defined by a user, or automatically. The analysis may consequently be performed more efficiently in the operation S120. If the extent of the X-ray image(s) is defined by a user, the system 200 illustrated in Fig. 2 may also include a display such as the monitor 220 illustrated in Fig. 2, or another display such as a display of a console or desktop computer or tablet, and a user input device such as a keyboard, a touch screen, a pointing device such as a mouse (not illustrated in Fig. 2). The processor(s) 120 receive user input from the display and/or user input device defining an extent of the X-ray image(s) 120 displayed on the display that are to be analysed in the operation S120. Subsequently, the analysis to determine the distribution of the stent struts 150 along the axis 160 of the lumen 140 is only performed within the user-defined extent.

In one example, the extent of the X-ray image(s) 120 is defined automatically using a neural network. A neural network such as YOLO, or alternatively a U-Net, may be used for this purpose. The YOLO neural network is described in a document by Redmon, J., et al. entitled "You Only Look Once: Unified, Real-Time Object Detection", published as arXiv: 1506.02640. The YOLO neural network generates bounding boxes around objects in inputted images, and may be used to identify the stent in the X-ray image(s) 120. Subsequently, the analysis to determine the distribution of the stent struts 150 along the axis 160 of the lumen 140, is only performed within the automatically-defined extent.

In another example, the extent of the X-ray image(s) 120 within which the X-ray image data is analysed in the operation S120 may be defined automatically by detecting the lumen in the X-ray image(s) 120. This may be performed using a feature detection algorithm to identify either a guidewire within the lumen, or a previously-injected contrast agent in the lumen, for example. Subsequently, the longitudinal extent of the lumen may be identified in the X-ray image(s) 120. The analysis to determine the distribution of the stent struts 150 along the axis 160 of the lumen 140, may then be performed only along the automatically-defined longitudinal extent of the lumen. The analysis may for example be carried out only within an area defined by the detected lumen, or within an area defined by a track along the lumen with a predetermined width. This procedure may assist in finding the stent more quickly, and is particularly beneficial when only a portion of the stent has been deployed.

Thus, in accordance with these examples, the one or more processors 110 are further configured to:
- receive input defining an extent of the one or more X-ray images 120 to be analysed; and
- analyse the X-ray image data by analysing the X-ray images 120 only within the defined extent.

In some examples, the system 100 may be used during deployment of a stent in order to determine an expected extent of the stent along the lumen. A physician typically deploys a stent such that it covers a planned length of a lumen. In the example of vascular stents, a stent may be deployed such that it covers a stenosed, i.e. narrowed, portion of the vessel. However, as mentioned above, due to their flexible nature, various factors can affect the deployed length of stents. For example, the rate at which the stent is deployed may affect whether longitudinal compressive or extensive deformations are introduced to the stent. The deployed length of a self-expanding stent may also be controlled to some extent by applying a compressive force to the stent via the proximal end of the plunger of the stent deployment device whilst withdrawing the stent's outer catheter. In these examples, the received X-ray image data that is received in the operation S110 represents one or more X-ray images 120 of a stent 130 within a lumen 140 during deployment of the stent 130 within the lumen 140, and the stent 130 includes a deployed portion and an un-deployed portion. The one or more processors 110 of the system are configured to:
- compute an expected post-deployment length of the un-deployed portion, based on the distribution of the stent struts 150 in the deployed portion; and
- indicate, in the displayed one or more X-ray images 120, an expected extent of the deployed stent 130 based on the computed expected post-deployment length of the un-deployed portion.

The expected extent of the deployed stent may for example be indicated by providing a marker indicating the expected end location of the stent, or alternatively displayed as a numerical value indicating how much more of the stent is yet to be deployed. By indicating the expected extent of the deployed stent 130 in this manner, a physician may determine whether there is a sufficient amount of the stent in the un-deployed portion to cover the desired portion of the lumen, and likewise, whether there is too much of the stent in the un-deployed portion. The physician may consequently adjust the pressure on the plunger of the stent deployment device in order to ensure that the correct portion of the lumen is covered, or alternatively re-cover the stent and then repeat its deployment.

In these examples, the un-deployed portion represents the remaining portion of the stent within the stent deployment device. Self-expanding stents are typically deployed from a catheter, and upon exiting the catheter, the stent expands radially to support the lumen. Since the distribution of stent struts 150 in the deployed portion is known, the number of deployed stent struts is known. Consequently, the number of stent struts that remain in the stent deployment device can be calculated based on the type of stent undergoing deployment. The expected post-deployment length may thus be calculated by extrapolating the expected end location of the stent based on the remaining number of stent struts remaining in the stent deployment device, and the nominal deployed length of the stent.

In these examples, the expected post-deployment length of the un-deployed portion may be determined by accessing a database. The database may include information such as the nominal deployed length of the stent, and the expected distribution of the stent struts 150 along the axis 160 of the lumen 140, for one or more stents with their nominal deployed length. The database may also include an error margin for each distribution in order to allow for inter-user variations. The inter-user variations may be provided for a selection of different types of stenoses. This allows the expected post-deployment length to be computed whilst accounting for such inter-user variations. The database may also include maximum and/or minimum recommended length of the stent based on manufacturer-recommended limits on the extensive or compressive forces that may be applied to the stent.

Thus, in these examples, the one or more processors 110 compute the expected post-deployment length of the un-deployed portion, by accessing a database of expected distributions of the stent struts 150 along the axis 160 of the lumen 140.

The maximum and/or minimum recommended length of the stent may also be displayed, and thereby used to indicate to a physician whether the stent can be positioned in the target location within the manufacturer-recommended limits.

In one example, a warning signal may be provided if the computed expected post-deployment length of the un-deployed portion is insufficient to overlap the desired extent. In some examples, different warnings may be provided in order to e.g. guide a physician to slightly deviate from the current deployment procedure by enforcing more or less compression on the stent, or alternatively to dramatically deviate from the current deployment procedure in order to deploy the stent in the target location whilst remaining within the mechanically-recommended bounds of the stent. In so doing, the physician may be alerted to adjust the manner in which the stent is deployed in order to achieve the desired coverage of the lumen.

In this example, the one or more processors 110 receive input indicative of a desired extent of the lumen 140 to be overlapped by the stent 130; and generate a warning signal if the computed expected post-deployment length of the un-deployed portion is insufficient to overlap the desired extent. The input indicative of a desired extent of the lumen 140 to be overlapped by the stent 130 may be inputted by a user, for example via the aforementioned user input device, or generated automatically by a therapy planning system. The therapy planning system may for example compute a length of the lumen that is to be covered by the stent based on the X-ray image(s) 120, and use this length as the value of the desired extent of the lumen 140 to be overlapped by the stent 130. The planning system may compute the length of the lumen that is to be covered by the stent based further on imaging data generated by an intraluminal imaging device, such as for example the IVUS imaging device 230 illustrated in Fig. 2, or another intraluminal imaging device, such as an OCT imaging device. The system 100 may also output one or more recommended follow-up actions, such as intravascular checks based on the distributions of the stent struts 150 in the deployed stent.

In another example, a computer-implemented method of identifying deformations of a deployed stent 130, is provided. The method includes:
- receiving X-ray image data representing one or more X-ray images 120 of a deployed stent 130 within a lumen 140, the stent 130 including a plurality of stent struts 150;
- analysing the X-ray image data to determine a distribution of the stent struts 150 along an axis 160 of the lumen 140; and
- identifying one or more longitudinally-deformed portions of the stent 130 based on a density of the determined distribution of the stent struts 150 along the axis of the lumen 140.

The computer-implemented method may also include one or more additional operations described above in relation to the system 100. The method may also be provided as a computer program product that includes instructions which when executed by one or more processors 110, cause the one or more processors to carry out the method.
The above examples are to be understood as illustrative of the present disclosure, and not restrictive. Further examples are also contemplated. For instance, the examples described in relation to the system 100, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for identifying deformations of a deployed stent, the system comprising one or more processors (110) configured to:
receive (SI10) X-ray image data representing one or more X-ray images (120) of a deployed stent (130) within a lumen (140), the stent including a plurality of stent struts (150);
analyse (S120) the X-ray image data to determine a distribution of the stent struts (150) along an axis (160) of the lumen (140); and
identify (S130) one or more longitudinally-deformed portions (170, 180) of the stent based on a density of the determined distribution of the stent struts (150) along the axis (160) of the lumen (140).

2. The system according to claim 1, wherein the one or more processors (110) are configured to identify the one or more longitudinally-deformed portions of the stent by:
displaying the one or more X-ray images (120), and indicating, in the displayed one or more X-ray images (120), the one or more longitudinally-deformed portions of the stent (130).

3. The system according to claim 1 or claim 2, wherein the one or more processors (110) are configured to identify the one or more longitudinally-deformed portions of the stent (130) by:
comparing the density of the determined distribution of the stent struts (150) along the axis (160) of the lumen (140), to one or more threshold density values; and
assigning a marker (190) indicative of the one or more threshold density values to one or more corresponding portions of the stent (130).

4. The system according to any one of claims 1 to 3, wherein the one or more processors (110) are further configured to:
i) compute the density of the determined distribution of the stent struts (150) along the axis (160) of the lumen (140) in relation to an expected density for the stent (130); and
identify the one or more longitudinally-deformed portions of the stent (130), by displaying the computed density as a proportion of the expected density;
and/or
ii) compute the density of the determined distribution of the stent struts (150) along the axis (160) of the lumen (140);
determine an actual length of the stent (130) based on the density; and
identify the one or more longitudinally-deformed portions of the stent (130), by displaying the actual length as a proportion of an expected length of the stent (130).

5. The system according to any one of claims 2 to 4, wherein:
the received X-ray image data represents one or more X-ray images (120) of a stent (130) within a lumen (140) during deployment of the stent (130) within the lumen (140);
wherein the stent (130) comprises a deployed portion and an un-deployed portion; and
wherein the one or more processors (110) are further configured to:
compute an expected post-deployment length of the un-deployed portion, based on the distribution of the stent struts (150) in the deployed portion; and
indicate, in the displayed one or more X-ray images (120), an expected extent of the deployed stent (130) based on the computed expected post-deployment length of the un-deployed portion.

6. The system according to claim 5, wherein the one or more processors (110) are configured to compute the expected post-deployment length of the un-deployed portion, by accessing a database of expected distributions of the stent struts (150) along the axis (160) of the lumen (140).

7. The system according to claim 5 or claim 6, wherein the one or more processors (110) are further configured to:
receive input indicative of a desired extent of the lumen (140) to be overlapped by the stent (130); and
generate a warning signal if the computed expected post-deployment length of the un-deployed portion is insufficient to overlap the desired extent.

8. The system according to claim 1, wherein the one or more processors (110) are configured to analyse the X-ray image data by:
applying a spatial filter to the one or more X-ray images (120), and estimating a density of the distribution of the stent struts (150) along the axis (160) of the lumen (140) by fitting a stent model to the filtered one or more X-ray images; or
estimating a density of the distribution of the stent struts (150) along the axis (160) of the lumen (140) by applying one of more image templates representing a predetermined distribution of stent struts (150), to the one or more X-ray images (120); or
inputting the one or more X-ray images (120) into a neural network (200) trained to predict a stent strut density distribution from inputted X-ray images (120).

9. The system according to claim 8, wherein the neural network is trained to predict a stent strut density distribution from inputted X-ray images (120), by:
receiving X-ray training image data representing a plurality of X-ray images (120) including a stent (130);
receiving ground truth data representing a labelled strut density along an axis of the stent (130); and
for a plurality of the X-ray images (120):
inputting the X-ray training image data into the neural network; and
adjusting parameters of the neural network based on a loss function representing a difference between a predicted strut density along an axis of the stent (130) that is predicted by the neural network, and the ground truth data for the stent (130).

10. The system according to any previous claim, wherein the one or more processors (110) are further configured to:
receive input defining an extent of the one or more X-ray images (120) to be analysed; and
analyse the X-ray image data by analysing the X-ray images (120) only within the defined extent.

11. The system according to any previous claim, wherein the one or more longitudinally-deformed portions comprise one or more longitudinally-compressed portions of the stent (130) and/or one or more longitudinally-extended portions of the stent (130).

12. The system according to any previous claim, wherein the one or more X-ray images (120) represented in the X-ray image data include an interventional device comprising a plurality of fiducial markers having a predetermined separation; and
wherein the one or more processors (110) are further configured to:
determine a scale factor for the one or more X-ray images (120) based on a measured separation between the plurality of fiducial markers in the one or more X-ray images (120), and the predetermined separation; and
determine the density of the determined distribution of the stent struts (150) along the axis (160) of the lumen (140) based on the scale factor.

13. The system according to claim 12, wherein the interventional device comprises a stent deployment device.

14. A computer-implemented method of identifying deformations of a deployed stent (130), the method comprising:
receiving X-ray image data representing one or more X-ray images (120) of a deployed stent (130) within a lumen (140), the stent (130) including a plurality of stent struts (150);
analysing the X-ray image data to determine a distribution of the stent struts (150) along an axis (160) of the lumen (140); and
identifying one or more longitudinally-deformed portions of the stent (130) based on a density of the determined distribution of the stent struts (150) along the axis of the lumen (140).

15. A computer program product comprising instructions which when executed by one or more processors (110), cause the one or more processors to carry out the method according to claim 14.
